# FASCICULE DE BREVET EUROPEEN

(11) **EP 2 395 863 B1**
(45) Date de publication et mention de la délivrance du brevet: **07.05.2014**
(21) Numéro de dépôt: 10701719.6
(22) Date de dépôt: 06.01.2010
(51) Int. Cl.: A41D 31/00, A61F 13/06, A61F 13/10, B29C 51/08, B32B 5/24, B32B 38/12, A41D 13/06, A41D 13/08, B29L 31/48, B29L 22/02, B29C 65/52, B29C 65/48, B29C 35/02, B29K 27/06, B29K 83/00, B29L 7/00, B29C 51/10

(54) **PROCÉDÉ DE FABRICATION D'UN DISPOSITIF DE PROTECTION D'UNE ZONE DU CORPS HUMAIN**
VERFAHREN ZUR HERSTELLUNG EINER VORRICHTUNG FÜR DEN SCHUTZ EINES BEREICHS DES MENSCHLICHEN KÖRPERS
METHOD FOR PRODUCING A DEVICE FOR PROTECTING AN AREA OF THE HUMAN BODY

(30) Priorité: 13.02.2009 FR 0900665
(43) Date de publication de la demande: 21.12.2011
(73) Titulaire: Millet Innovation, 26270 Loriol sur Drome (FR)
(72) Inventeur: GRANGE, Odile, F-26400 Allex (FR); MILLET, Damien, F-26000 Valence (FR)
(74) Mandataire: Marchand, André
(86) Numéro de dépôt international: PCT/IB2010/000033
(87) Numéro de publication internationale: WO 2010/092445

(56) Documents cités:
- WO-A-01/74566
- WO-A-2008/128206
- FR-A- 2 793 678
- FR-A- 2 892 298
- US-A- 5 670 232

## Description

La présente invention concerne un procédé de fabrication d'un dispositif de protection comprenant une pièce de tissu et un module en gel viscoélastique collé sur la pièce de tissu.

Des dispositifs de protection du type précité sont généralement prévus pour protéger des zones du corps humain, par exemple des zones souffrant d'escarres, d'ampoules, de crevasses, ou pour protéger des zones saines contre les chocs, contre l'échauffement par frottement, etc. Ils sont généralement lavables et réutilisables.

Un procédé de fabrication classique d'un dispositif de protection du type précité comprend généralement les étapes suivantes :
- dépôt d'un composé réticulable dans un moule,
- réticulation du composé pour obtenir un module en gel viscoélastique,
- démoulage du module,
- découpe d'une pièce de tissu,
- collage du module sur la pièce de tissu, et
- optionnellement, couture ou soudure de bords de la pièce de tissu, pour former une orthèse pouvant être enfilée sur la zone du corps à protéger (main, pied, bras, jambe, etc.).

Ce procédé de fabrication comprend généralement des étapes manuelles, car la texture gélatineuse des modules en gel viscoélastique les rend impropres à être manipulés par des machines. Le temps de réticulation des gels viscoélastiques représente une contrainte supplémentaire faisant obstacle à la prévision d'une ligne de fabrication en série.

Certaines étapes de ce procédé peuvent être rationalisées dans le cadre d'une production industrielle, par exemple en réalisant simultanément une pluralité de modules en gel viscoélastique dans des moules à cavités multiples. Toutefois, les étapes de démoulage, de transport puis de collage des modules sur des pièces de tissu restent difficile à automatiser.

Il peut ainsi être souhaité de prévoir un procédé de fabrication qui permette de réduire de façon significative le prix de revient de tels dispositifs, en rationalisant les étapes de fabrication et de collage sur des pièces de tissu des modules en gel viscoélastique.

Les demandes internationales WO 2000/71066 et WO 2007/045737 décrivent des procédés de fabrication de dispositifs de protection du type précité. Elles enseignent de recouvrir une bande de tissu d'une couche de gel viscoélastique également en forme de bande. La bande de tissu est ensuite rabattue bord à bord longitudinalement, et les bords se trouvant en regard sont cousus ou soudés. La bande est ensuite découpée en sections de longueurs déterminées pour former des manchons de protection du doigt ou de l'orteil appelés "digitubes" (marque déposée). De tels procédés de fabrication concernent donc spécifiquement la fabrication de manchons de protection et ne sont pas généralisables à la fabrication industrielle de dispositifs de protection de forme quelconque. La demande internationale WO2008/128206 présente un procédé de fabrication d'un dispositif de protection comprennant une pièce de tissu et un module en gel viscoélastique dont le module est réticulé après que la pièce de tissu soit déposée sur le composé non réticulé.

Des modes de réalisation de l'invention concernent un procédé de fabrication collective d'un dispositif de protection comprenant une pièce de tissu et un module en gel viscoélastique collé à la pièce de tissu, comprenant les étapes consistant à former une pluralité d'alvéoles dans une bande support en un matériau déformable, remplir les alvéoles avec un composé non réticulé, réticuler le composé pour obtenir des modules en gel viscoélastique dans les alvéoles, déposer sur chaque module une substance adhésive, et déposer une bande de tissu sur la bande support pour obtenir une bande composée finale dans laquelle les modules en gel viscoélastique présents dans les alvéoles sont collés à la bande de tissu par la substance adhésive.

Selon un mode de réalisation, le procédé comprend, entre les étapes de formation des modules et de dépôt d'une substance adhésive sur les modules, les étapes consistant à déposer sur la bande support un film de protection, pour obtenir une bande composée intermédiaire dans laquelle les modules en gel viscoélastique présents dans les alvéoles sont couverts par le film de protection, et enrouler la bande composée intermédiaire de manière à former un rouleau de bande composée intermédiaire.

Selon un mode de réalisation, le film de protection est déposé sur la bande support avant que le composé dans les alvéoles soit entièrement réticulé, et le procédé comprend une étape de stockage du rouleau de bande composée intermédiaire pendant laquelle la réticulation du composé peut se poursuivre.

Selon un mode de réalisation, le procédé comprend, avant l'étape de dépôt de la substance adhésive sur les modules en gel viscoélastique, une étape consistant à dérouler le rouleau de bande composée intermédiaire et à retirer le film de protection.

Selon un mode de réalisation, le procédé comprend une étape consistant à former un rouleau avec la bande composée finale.

Selon un mode de réalisation, la substance adhésive est une colle polymérisable, et le procédé comprend une étape de stockage du rouleau de bande composée finale pendant laquelle la substance adhésive se polymérise ou continue de se polymériser.

Selon un mode de réalisation, le procédé comprend une étape consistant à séparer la bande support de la bande de tissu sans décoller les modules en gel viscoélastique de la bande de tissu.

Selon un mode de réalisation, le procédé comprend une étape de découpe de la bande de tissu, pour obtenir une pièce de tissu comprenant au moins un module en gel viscoélastique.

Selon un mode de réalisation, le procédé comprend une étape de pliage de la pièce de tissu selon un axe de pliage, et des étapes de couture ou de soudure de bords de la pièce de tissu, pour former un dispositif de protection pouvant être enfilé sur une partie du corps humain.

Selon un mode de réalisation, le procédé est appliqué à la fabrication d'un dispositif de protection du pied et comprend : une étape de découpe dans la bande de tissu d'une pièce de tissu comprenant quatre grandes branches, dont deux à gauche d'un axe de pliage et deux à droite de l'axe de pliage, entre deux grandes branches, deux petites branches tronquées agencées à gauche et à droite de l'axe de pliage et orientées vers l'axe de pliage, comportant deux bords se rejoignant sur l'axe de pliage, et entre deux autres grandes branches, deux petites branches tronquées agencées à gauche et à droite de l'axe de pliage et orientées vers l'axe de pliage, comportant deux bords se rejoignant sur l'axe de pliage ; une étape de pliage de la pièce de tissu de manière que les petites branches se superposent ; et une étape de couture ou de soudure des petites branches se superposant, pour former un module de protection du talon, les grandes branches étant destinées à être reliées deux à deux par des moyens de liaison, pour former des bretelles de maintien du module de protection du talon.

Selon un mode de réalisation, le procédé est appliqué à la fabrication d'un dispositif de protection de la main et comprend : une étape de découpe dans la bande de tissu d'une pièce de tissu présentant deux parties symétriques relativement à un axe de pliage ; une étape de pliage de la pièce de tissu en sorte que les deux parties soient l'une au-dessus de l'autre ; et une étape de couture ou de soudure pour relier deux à deux les bords se superposant des deux parties, de sorte que le dispositif présente des bords non cousus ou non soudés formant des ouvertures permettant le passage du poignet et des doigts.

Selon un mode de réalisation, la bande support est en polychlorure de vinyle.

Selon un mode de réalisation, le procédé comprend les modules sont en gel silicone.

Selon un mode de réalisation, le procédé comprend le tissu est du polyamide élasthanne.

Un exemple de réalisation d'un procédé de fabrication selon l'invention sera décrit à titre non limitatif dans ce qui suit, en relation avec les figures jointes parmi lesquelles :
- les figures 1A, 1B sont des vues en coupe illustrant schématiquement des étapes du procédé de fabrication,
- la figure 2A est une vue de dessus d'une bande support au terme d'une étape S2 représentée sur la figure 1A,
- la figure 2B est une vue de dessus de la bande support au terme d'une étape S7 représentée sur la figure 1B,
- la figure 3A est une vue de dessus d'une bande de tissu comportant des modules en gel viscoélastique, réalisée au moyen du procédé précité,
- la figure 3B représente la bande de tissu après découpe de celle-ci, de manière à obtenir des dispositifs de protection non assemblés,
- les figures 4A, 4B représentent sous forme non assemblée et sous forme assemblée un exemple de dispositif de protection, et
- les figures 5A, 5B représentent sous forme non assemblée et sous forme assemblée un autre exemple de dispositif de protection.

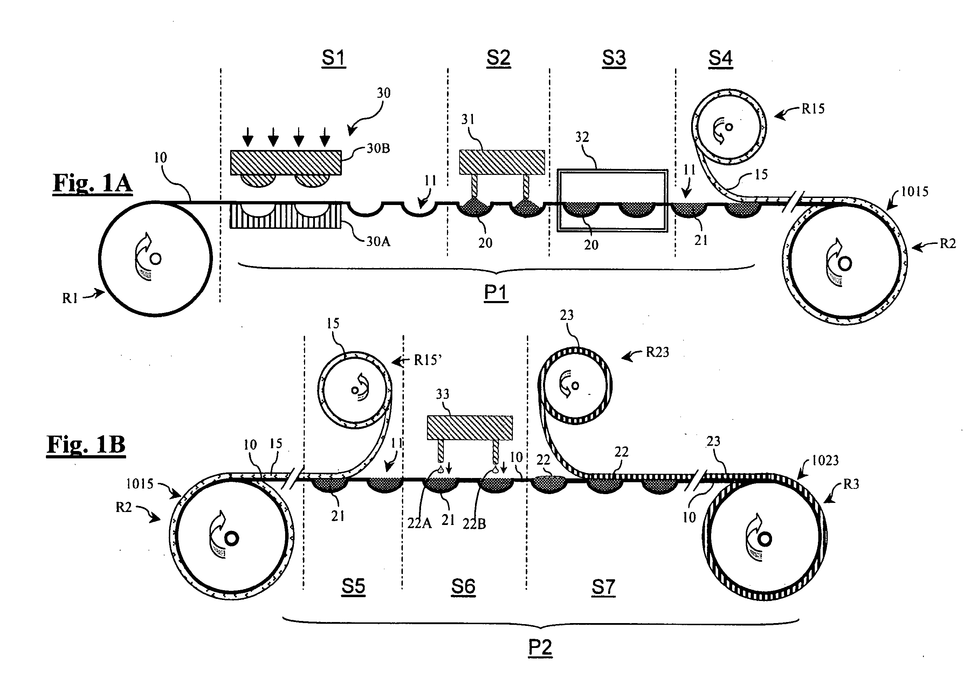

Les figures 1A, 1B représentent des étapes d'un mode de réalisation d'un procédé de fabrication collective de dispositifs de protection selon l'invention. Le procédé comprend des phases P1, P2 et P3. La phase P1, illustrée sur la figure 1A, comprend des étapes de formation de modules en gel viscoélastique 21 dans des alvéoles 11 d'une bande support 10. La phase P2, illustrée sur la figure 1B, comprend une étape de collage des modules 21 sur une bande de tissu 23, sans extraire les modules des alvéoles 11. La phase P3 comprend une étape de séparation de la bande support 10 relativement à la bande de tissu 23, sans décoller les modules 21, et une étape de découpe de la bande de tissu pour obtenir des dispositifs de protection non assemblés.

La phase P1 est mise en oeuvre dans une première ligne de fabrication représentée schématiquement sur la figure 1A. A une première extrémité de la ligne de fabrication est agencé un rouleau R1 de la bande support 10. Dans un mode de réalisation, la bande support 10 est en un polymère thermoplastique, par exemple du PVC (polychlorure de vinyle). Elle présente par exemple une épaisseur de l'ordre de 1 à 2 dixièmes de millimètre. Le rouleau R1 est déroulé pas à pas et la bande support 10 se voit appliquer des étapes de traitement S1 à S4 au fur et à mesure de sa progression. Un second rouleau R2 est formé avec la bande support 10, à l'autre extrémité de la ligne de fabrication.

A l'étape S1, une portion de la bande support 10 est déformée à chaud par un outil 30, de manière à former les alvéoles 11 susmentionnées. Dans un mode de réalisation, l'outil 30 est une presse de thermoformage qui comprend une partie inférieure 30A et une partie supérieure 30B. La partie 30A comporte des empreintes négatives en forme d'alvéoles et la partie 30B comporte des empreintes positives dont la forme correspond à celle des alvéoles de l'empreinte négative. Dans une variante de réalisation, un système d'aspiration peut également être utilisé pour thermoformer la bande support dans une empreinte négative ayant la forme désirée.

A chaque progression pas à pas de la bande support 10, un nouveau groupe d'alvéoles 11 est formé. En aval de la presse de thermoformage, la bande support 10 présente une suite ininterrompue d'alvéoles 11 agencées en série dans le sens de défilement de la bande support. L'espacement entre les alvéoles est choisi en fonction de la forme et de la taille des dispositifs de protection à réaliser, dont des exemples seront décrits plus loin.

Dans un souci de simplification du dessin, la presse 30 représentée sur la figure 1A ne comporte que deux alvéoles. En pratique, un plus grand nombre d'alvéoles peut être réalisé simultanément. De plus, outre la formation d'alvéoles 11 en série dans le sens de défilement de la bande support, la presse 30 pourrait être conformée de manière à réaliser également des alvéoles en parallèle relativement au sens de défilement de la bande support. Cela peut par exemple être le cas lorsque des dispositifs de protection à réaliser doivent comporter deux modules en gel viscoélastique, voire plus, agencés côte à côte. Toutefois, des modules destinés à être intégrés dans le même dispositif de protection pourraient aussi être agencés en série sur la bande.

A l'étape S2, les alvéoles 11 passent sous un distributeur-doseur 31 comprenant une ou plusieurs buses de dispensation d'un composé réticulable 20. Le composé 20 est déposé dans les alvéoles 11, chaque buse de dispensation assurant le remplissage d'une alvéole. Le composé est par exemple un mélange fluide d'huiles silicones réticulables.

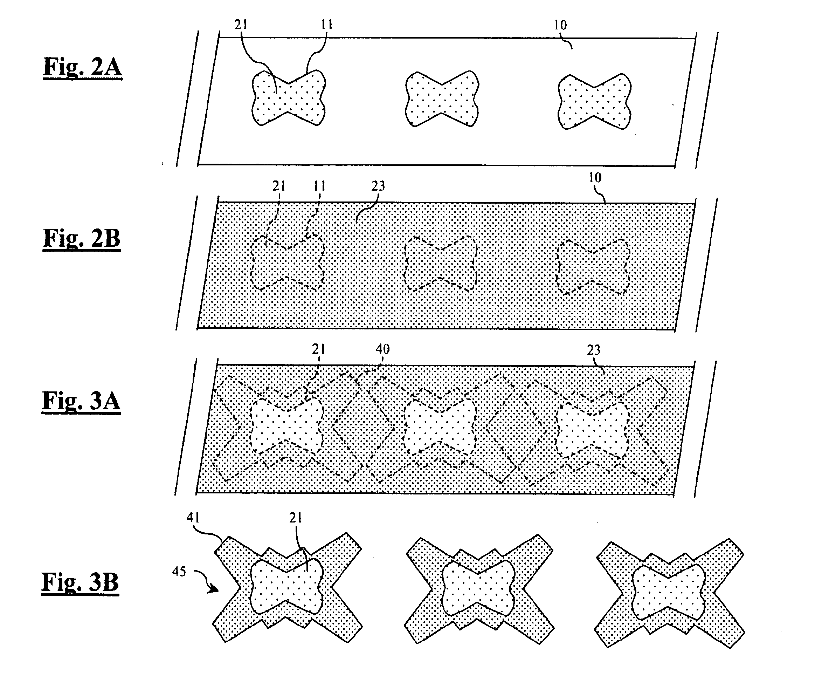

A l'étape S3, les alvéoles 11 remplies du composé réticulable 20 passent dans un tunnel de chauffe 32 pour réticulation du composé 20. La température dans le tunnel de chauffe est par exemple de l'ordre de 40 à 65°C. La durée du processus est par exemple de quelques minutes. Au sortir du tunnel de chauffe, le composé 20 est au moins partiellement réticulé et les alvéoles 11 contiennent ainsi les modules en gel viscoélastique 21 susmentionnés, par exemple du gel silicone. A titre d'illustration, la figure 2A est une vue de dessus de la bande support 10 et des alvéoles 11 contenant les modules 21.

A l'étape S4, un film de protection 15 est appliqué sans collage sur la bande support 10, de manière à recouvrir les modules 21. Le film de protection 15 est par exemple fourni par un rouleau R15 qui est déroulé pas à pas en synchronisation avec la progression de la bande support. Le film 15 adhère naturellement mais sans excès sur les modules 21. Le film 15 est par exemple un film en polyéthylène du type utilisé dans l'industrie alimentaire. On obtient ainsi une bande composée intermédiaire 1015 comprenant la bande support 10 et le film 15, dans laquelle les modules 21 présents dans les alvéoles sont recouverts par le film 15. La bande composée 1015 est enroulée autour d'un axe pour former le rouleau R2, qui peut être stocké en attendant la phase P2. Ainsi, si les modules 21 ne sont pas complètement réticulés lorsque le rouleau R2 est formé, ils peuvent continuer à réticuler à température ambiante pendant le stockage du rouleau R2. Un stockage à température forcée peut également être envisagé, selon les cadences de production visées et les moyens de stockage disponibles.

Comme cela est indiqué ci-dessus, la phase P1 est mise en oeuvre pas à pas et le défilement de la bande support 10 est arrêté à chaque étape de thermoformage S1, de remplissage S2, et de chauffage S3. Les étapes S1, S2, S3 peuvent être initiées simultanément puisqu'elles sont appliquées à des portions différentes de la bande support, l'étape la plus longue à réaliser imposant la vitesse de défilement pas à pas. Il appartiendra à l'homme de l'art d'optimiser chaque étape de manière que la vitesse de défilement soit maximale ou adaptée au rythme de production souhaité. Notamment, le fait de ne viser qu'une réticulation partielle des modules 21 au cours de l'étape S3 permet de ne pas pénaliser la vitesse de défilement de la bande support. A titre d'exemple, la vitesse moyenne (pauses incluses) de défilement de la bande support 10 peut être typiquement de l'ordre 0,5 à 2 mètres par minute.

La phase P2 est mise en oeuvre dans une seconde ligne de fabrication représentée schématiquement sur la figure 1B. Le rouleau R2 de bande composée intermédiaire 1015 est agencé à une première extrémité de la seconde ligne de fabrication. Le rouleau R2 est déroulé et la bande support 10 se voit appliquer des étapes de traitement S5 à S7 au fur et à mesure de sa progression. Un troisième rouleau R3 est formé avec la bande support 10, à l'autre extrémité de la seconde ligne de fabrication.

A l'étape S5, le film de protection 15 est retiré de la bande support 10. Le film 15 est par exemple enroulé autour d'un axe pour former un rouleau R15' qui pourra ensuite être recyclé. La face supérieure des modules 21 dans les alvéoles 11 se trouve donc de nouveau à l'air libre.

A l'étape S6, les modules 21 passent sous un distributeur-doseur 33 comprenant une ou plusieurs buses de dispensation au moyen desquelles une colle polymérisable 22 est déposée sur les modules 21. La colle 22 est par exemple formée par deux composants fluides 22A, 22B distribués chacun par une buse du distributeur-doseur, qui polymérisent lorsqu'ils sont en contact. Une fine pellicule de colle 22 est ainsi déposée sur les modules 21. Dans une variante de réalisation de l'étape S6, une colle monocomposant est fournie par une seule buse du distributeur-doseur et est ensuite étalée à la surface des modules 21 au moyen de rouleaux d'enduction qui assurent un contact roulant sur les modules 21 avec application d'une légère pression. Dans une autre variante de réalisation, une machine dépose à la surface de chaque module 21 une pellicule adhésive 22 qui est adhésive sur ses deux faces. Contrairement à la phase P1, la phase P2 peut être mise en oeuvre avec défilement continu de la bande support 10, le dépôt de colle 22 ne nécessitant pas l'arrêt de la bande support.

A l'étape S7, la bande de tissu 23 susmentionnée est appliquée sur la bande support 10. La bande de tissu 23 est par exemple fournie par un rouleau R23 qui est déroulé en synchronisation avec la progression de la bande support. Le tissu 23 est par exemple un polyamide élasthanne. Au moment de l'application de la bande de tissu 23 sur la bande support 10, les modules 21 se collent sur le tissu 23 grâce à la fine couche ou pellicule de colle 22, sans que le tissu se colle à la bande support 10. On obtient ainsi une bande composée finale 1023 comprenant la bande support 10 et le tissu 23, dans laquelle les modules 21 présents dans les alvéoles sont collés au tissu 23. A titre d'illustration, la figure 2B montre par une vue de dessus l'aspect de la bande support 10 recouverte par le tissu 23. Les alvéoles 11 et les modules 21 se trouvent sous le tissu 23 et sont représentés en traits pointillés.

La bande composée 1023 est enroulée pour former le rouleau R3, qui peut être stocké en prévision de la phase P3. Il est à noter qu'à l'instar du gel viscoélastique 20 lorsque le rouleau R2 est formé, la colle 22 peut ne pas être complètement polymérisée lorsque le rouleau R3 est formé. La colle 22 peut continuer à polymériser à température ambiante ou forcée pendant le stockage du rouleau R3.

Au cours de la phase P3, le rouleau R3 est déroulé, la bande support 10 est séparée de la bande de tissu 23 pour obtenir une bande de tissu 23 sur laquelle sont collés les modules 21, comme illustré sur la figure 3A. La bande de tissu 23 est ensuite découpée selon un motif déterminé 40 pour obtenir des pièces de tissu 41 du type représenté sur la figure 3B, comportant chacune au moins un module en gel viscoélastique 21. Chaque pièce de tissu 41 et son module 21 forme un dispositif de protection 45 non assemblé. Dans une variante de réalisation, la bande de tissu 23 est découpée avant retrait de la bande support 10. La séparation de la bande support est ensuite faite pièce par pièce.

La phase P3 peut être automatisée ou manuelle. Elle peut par exemple être exécutée par une machine de séparation qui enroule la bande support 10 autour d'un axe pour former un rouleau recyclable, et qui assure ensuite la découpe du tissu, par exemple au moyen d'un faisceau laser.

Dans une variante de réalisation de la phase P3, la machine de séparation et de découpe est agencée en ligne avec les machines utilisées pour mettre en oeuvre la phase P2. Dans ce cas, il n'est pas nécessaire de former le rouleau R3. Dans une autre variante de réalisation, le rouleau R3 est remis à une entreprise de confection qui assure l'extraction les modules des alvéoles par déroulement de la bande de tissu 23 sur laquelle les modules sont collés, et la découpe de la bande de tissu.

La phase P3 peut être suivie d'une phase d'assemblage P4. On entend par "assemblage" le fait de conduire des étapes de couture ou de soudure de certains bords des pièces de tissu 41, pour former des dispositifs de protection assemblés pouvant être enfilés sur les zones du corps à protéger.

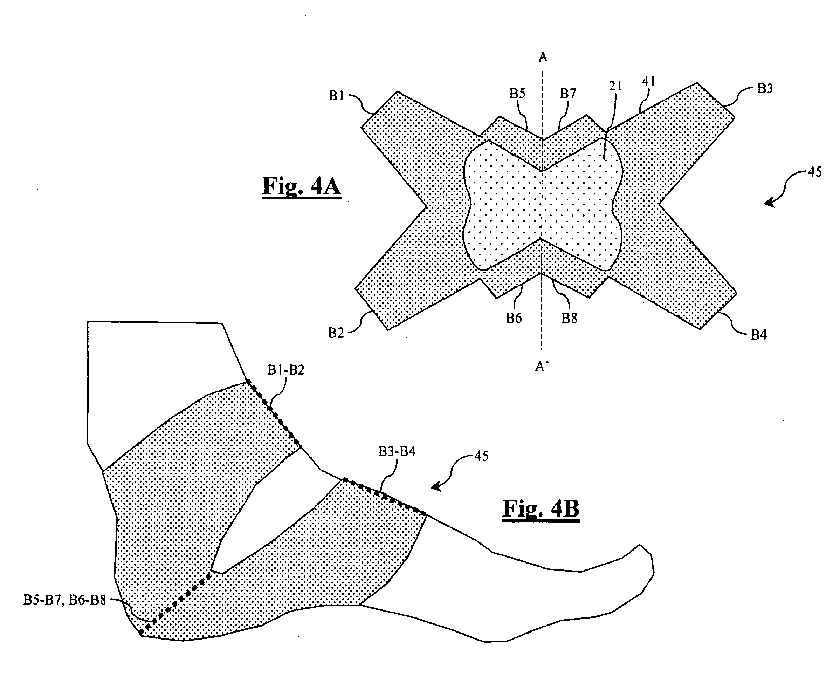

Les figures 4A, 4B représentent, respectivement sous forme non assemblée et sous forme assemblée, un exemple de réalisation d'un dispositif de protection 45 pouvant être fabriqué selon le procédé qui vient d'être décrit. Le dispositif 45 est prévu pour protéger le talon et permet par exemple de soigner les pathologies inflammatoires ou les hyperkératoses donnant naissance à des crevasses. Il comprend une pièce de tissu 41 et un module 21 en gel viscoélastique dont la forme ressemble à deux ailes de papillon. Dans un mode de réalisation, le module 21 est en un gel silicone très adhésif sur la peau.

La forme en ailes de papillon du module 21 est donc celle qui est retenue pour thermoformer les alvéoles de la bande support au cours de l'étape S1 de la phase P1 (Fig. 1A). Le module 21 est coulé en continu dans les alvéoles, comme indiqué, et est ensuite transféré et collé sur la bande de tissu 23 au cours de l'étape S7 de la phase P2 (Fig. 1B). La bande de tissu est ensuite découpée selon la forme représentée sur la figure 4A (ou Fig. 3A). Plus particulièrement, la pièce de tissu présente ici une forme en étoile symétrique selon un axe de pliage AA' qui comporte :
- quatre grandes branches tronquées B1, B2, B3, B4 agencées sensiblement en croix dont deux B1, B2 sont agencées à gauche de l'axe AA' et deux autres B3, B4 sont agencées à droite de l'axe AA',
- entre les branches tronquées B1, B3, deux petites branches tronquées B5, B7 orientées vers l'axe AA' dont les extrémités forment deux bords se rejoignant sur l'axe AA', l'un à gauche de l'axe AA' et l'autre à droite de l'axe AA', les deux petites branches formant ensemble un bord sensiblement en forme de "W" inversé,
- entre les branches B2, B4, deux petites branches tronquées B6, B8 orientées vers l'axe AA' mais de direction opposée à celle des branches B5, B7, dont les extrémités forment deux bords se rejoignant sur l'axe AA', l'un à gauche de l'axe AA' et l'autre à droite de l'axe AA', les deux petites branches formant ensemble un bord sensiblement en forme de "W".

L'assemblage du dispositif de protection 45 comprend tout d'abord une étape de pliage du tissu 41 selon l'axe AA', puis des étapes de couture ou soudure des extrémités des branches B5 et B7, B6 et B8 à proximité de leur bords, par exemple à environ 1 mm du module 21. Si une soudure est effectuée, l'excédent de tissu des branches peut être éliminé pendant l'opération. Si une couture est réalisée, l'excédent peut ensuite être enlevé par découpe.

Le dispositif peut ensuite être finalisé selon deux modes de réalisation. Dans un premier mode de réalisation, l'extrémité de la branche B1 est cousue ou soudée à l'extrémité de la branche B2 et l'extrémité de la branche B3 est cousue ou soudée à l'extrémité de la branche B4. Une fois le dispositif enfilé autour du pied, comme représenté sur la figure 4B, la couture ou la soudure B1-B2 s'étend sur la partie basse du tibia, la couture ou soudure B3-B4 s'étend sur la voussure du pied, et les coutures ou soudures B5-B7 et B6-B8 ceinturent le talon.

Dans un second mode de réalisation, les branches B1, B2, B3, B4 sont prévues sensiblement plus longues et ne sont pas cousues ou soudées. L'utilisateur peut ensuite nouer lui-même les extrémités des branches B1 et B2 et les extrémités des branches B3 et B4. D'autres moyens de liaison des extrémités des branches B1, B2 et B3, B4 pourraient aussi être envisagés, par exemple un matériau de type Velcro®.

En résumé, la zone cousue ou soudée du tissu qui incorpore le module 21 forme une coquille de protection du talon, et le dispositif 45 présente deux bretelles de maintien formées par les grandes branches de la pièce de tissu qui peuvent être cousues, soudées, ou attachées par un moyen de liaison temporaire, voire nouées par l'utilisateur.

Une telle structure de dispositif de protection permet un maintien excellent de la zone d'appui du talon et les deux bretelles assurent un maintien stable et équilibré du dispositif. La symétrie du dispositif permet également de s'affranchir de la nécessité de réaliser un dispositif pour pied gauche et un dispositif pour pied droit.

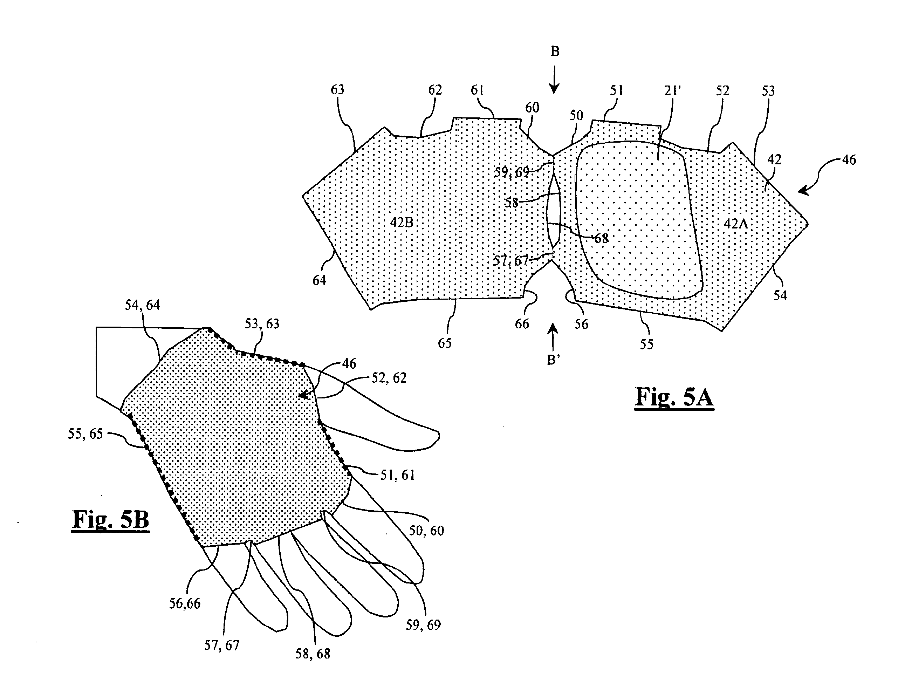

Les figures 5A, 5B représentent, respectivement sous forme non assemblée et sous forme assemblée, un autre exemple de dispositif de protection 46 pouvant être fabriqué selon le procédé décrit plus haut. Le dispositif 46 est prévu pour protéger la paume de la main. Il comporte une pièce de tissu 42 et un module 21' en gel viscoélastique collé sur la pièce de tissu. Le module 21' est ici de forme quadrilatère avec des angles arrondis, correspondant sensiblement à la forme de la paume de la main. La pièce de tissu 42 est coupée de manière à présenter deux partie 42A, 42B symétriques relativement à un axe de pliage BB'. La partie 42A, à droite de l'axe BB' sur la figure 5A, reçoit le module 21' et présente des bords 50, 51, 52, 53, 54, 55, 56, 57, 58, 59. La partie 42B est l'image de la partie 42A relativement à l'axe BB' en tant qu'axe de symétrie et présente des bords 60, 61, 62, 63, 64, 65, 66, 67, 68, 69. Les bords 57 et 67, ainsi que les bords 59 et 69 sont confondus et alignés avec l'axe BB', la pièce de tissu 42 n'étant pas découpée le long de ces bords. Ainsi, les bords 57 et 67, 59 et 69 relient les parties 42A, 42B avant couture ou soudure de celles-ci. Les bords 58, 68 sont obtenus en découpant une fente le long de l'axe BB', entre les bords 57, 67 et les bords 59, 69.

L'assemblage du dispositif 46 comprend une étape de pliage de la pièce 42 relativement à l'axe BB', de manière que les parties 42A, 42B soient l'une au-dessus de l'autre, et des étapes de couture ou de soudure des bords 51 et 61, 53 et 63, 55 et 65. Le dispositif peut ensuite être enfilé autour de la main comme représenté sur la figure 5B. Les bords non cousus 54, 64 forment une ouverture permettant le passage du poignet, les bords non cousus 52, 62 forment une ouverture permettant le passage du pouce, les bords non cousus 50, 60 forment une ouverture permettant le passage de l'index, les bords non cousus 58, 68 forment une ouverture permettant le passage du majeur et de l'annulaire, et les bords non cousus 56, 66 forment une ouverture permettant le passage de l'auriculaire.

Les maux à soigner évoqués ci-dessus pour la protection du talon peuvent également concerner la main et justifier l'utilisation du dispositif de protection 46. Celui-ci peut également être utilisé, par exemple, dans le cadre de la pratique sportive ou du jardinage. Ainsi, de nouveau, un simple pliage et quelques étapes de couture ou de soudure permettent la transformation d'un objet plan en un produit tridimensionnel bien adapté à la protection de la zone du corps visée.

Bien que le procédé décrit plus haut se prête bien à une fabrication industrielle des dispositifs de protection 45, 46, il sera noté que chacun de ces dispositifs forme également, en tant que tel, un objet innovant indépendamment de son procédé de fabrication.

D'autres modes de réalisation du procédé de fabrication selon l'invention peuvent permettent de réaliser d'autres types de modules en gel viscoélastique, concernant d'autres parties du corps. Il conviendra de noter la différence entre un procédé selon l'invention, dans lequel l'étape de couture ou de soudure du tissu est conduite après découpe du tissu, et un procédé du type décrit par les demandes WO 2000/71066 et WO 2007/045737, dans lequel l'étape de couture ou de soudure est au contraire effectuée avant découpe du tissu. Une autre différence tient au fait que le module en gel viscoélastique est en forme de bande continue dans les digitubes® selon WO 2000/71066 et WO 2007/045737, alors qu'un procédé selon l'invention permet de réaliser des modules distincts grâce aux alvéoles formées dans la bande support.

Il apparaîtra clairement à l'homme de l'art que divers autres modes de réalisation du procédé de fabrication selon l'invention peuvent être prévus. Par exemple, d'autres matériaux que du PVC pourraient être utilisés pour former la bande support 10. Le matériau thermoplastique formant la bande support doit de préférence présenter tout ou partie des propriétés suivantes :
- une mouillabilité appropriée, de manière que le composé liquide 20 se répande correctement dans les alvéoles au cours de l'étape S2,
- une bonne tenue en température pour passer le tunnel de chauffe 32 sans déformation,
- une bonne flexibilité mécanique pour s'enrouler sans pli lors de la formation du rouleau R2 ou R3,
- une faible adhésivité relative du gel viscoélastique dans les alvéoles 11, de manière que la force nécessaire à l'extraction des modules 21 des alvéoles 11 soit suffisamment faible pour ne pas décoller les modules 21 de la bande de tissu 23, et
- une bonne neutralité vis-à-vis du processus de réticulation. Ainsi, par exemple, un matériau comme le polyester peut inhiber le processus de réticulation de certaines huiles silicones.

Également, l'utilisation d'un matériau déformable à froid pourrait être envisagée pour réaliser la bande support et ses alvéoles.

L'homme de l'art notera également que les phases P1 et P2 illustrées sur les figures 1A, 1B pourraient être mises en oeuvre dans une seule ligne de fabrication. Dans un tel mode de réalisation, le film de protection 15 ne serait pas nécessaire, ni la formation du rouleau R2. Le fait que les phases P1, P2 ne soient pas mises en oeuvre dans une même ligne de fabrication offre toutefois des avantages en relation avec le temps de réticulation du gel. Ainsi, la mise en rouleau R2 de la bande support 10 permet de stocker celle-ci jusqu'à ce que les modules 21 soient complètement réticulés, comme cela a déjà été indiqué. Il suffit, au terme de l'étape S3, que les modules 21 soient suffisamment réticulés pour pouvoir être recouverts par le film de protection 15, mais il n'est pas nécessaire qu'ils soient entièrement réticulés. Leur réticulation peut se poursuivre pendant plusieurs heures, durant le stockage des rouleaux R2. A contrario, il est préférable que les modules 21 soient entièrement réticulés avant d'être soumis à l'étape de collage S7. Ainsi, la mise en ligne des phases P1, P2 nécessite d'allonger le tunnel de chauffe 32 (à cadence de fabrication constante) pour obtenir la complète réticulation des modules avant l'étape de collage S7, ce qui implique une consommation d'énergie plus importante en compensation du temps de réticulation dont on dispose dans le cas d'un stockage du rouleau R2.

Outre le gain en consommation d'énergie, le fait de scinder les phases P1, P2 en deux lignes de fabrication distinctes peut offrir d'autres avantages. Par exemple, il n'est pas nécessaire que les deux lignes de fabrication présentent la même vitesse de défilement et le même mode de défilement de la bande support (pas à pas pour la phase P1 et continu pour la phase P2). Également, la machine de collage 33 peut être utilisée pour d'autres applications pendant la fabrication de rouleaux R2 et le stockage de ceux-ci.

Des modes de réalisation du procédé de fabrication selon l'invention ont été initialement conçus pour réaliser des dispositifs de protection de zones du corps humain. Toutefois, il va de soi que l'invention n'est pas limitée à cette application. Des modes de réalisation de l'invention peuvent par exemple être prévus pour réaliser des dispositifs de protection du corps animal voire des dispositifs destinés à être agencés sur des objets inertes.

## Revendications

1. Procédé de fabrication collective d'un dispositif de protection (45, 46) comprenant une pièce de tissu (41, 42) et un module (21, 21') en gel viscoélastique collé à la pièce de tissu, **caractérisé en ce qu'**il comprend les étapes consistant à :
- former une pluralité d'alvéoles (11) dans une bande support (10) en un matériau déformable,
- remplir les alvéoles avec un composé non réticulé (20),
- réticuler le composé (20) pour obtenir des modules (21, 21') en gel viscoélastique dans les alvéoles,
- déposer sur chaque module (21, 21') une substance adhésive (22), et
- déposer une bande de tissu (23) sur la bande support (10) pour obtenir une bande composée finale (1023) dans laquelle les modules (21, 21') en gel viscoélastique présents dans les alvéoles (11) sont collés à la bande de tissu (23) par la substance adhésive (22).

2. Procédé selon la revendication 1 comprenant, entre les étapes de formation des modules (21, 21') et de dépôt d'une substance adhésive (22) sur les modules (21), les étapes consistant à :
- déposer sur la bande support (10) un film de protection (15), pour obtenir une bande composée intermédiaire (1015) dans laquelle les modules (21, 21') en gel viscoélastique présents dans les alvéoles (11) sont couverts par le film de protection (15),
- enrouler la bande composée intermédiaire (1015) de manière à former un rouleau (R2) de bande composée intermédiaire.

3. Procédé selon la revendication 2, dans lequel le film de protection (15) est déposé sur la bande support avant que le composé (20) dans les alvéoles soit entièrement réticulé, et comprenant une étape de stockage du rouleau (R2) de bande composée intermédiaire (1015) pendant laquelle la réticulation du composé (20) peut se poursuivre.

4. Procédé selon l'une des revendications 2 et 3, comprenant, avant l'étape de dépôt de la substance adhésive (22) sur les modules (21, 21') en gel viscoélastique, une étape consistant à dérouler le rouleau (R2) de bande composée intermédiaire (1015) et à retirer le film de protection (15).

5. Procédé selon l'une des revendications 1 à 4, comprenant une étape consistant à former un rouleau (R3) avec la bande composée finale (1023).

6. Procédé selon la revendication 5, dans lequel la substance adhésive (22) est une colle polymérisable, et comprenant une étape de stockage du rouleau (R3) de bande composée finale pendant laquelle la substance adhésive se polymérise ou continue de se polymériser.

7. Procédé selon l'une des revendications 1 à 6, comprenant une étape consistant à séparer la bande support (10) de la bande de tissu (23) sans décoller les modules (21, 21') en gel viscoélastique de la bande de tissu.

8. Procédé selon la revendication 7, comprenant une étape de découpe de la bande de tissu, pour obtenir une pièce de tissu (41, 42) comprenant au moins un module (21, 21') en gel viscoélastique.

9. Procédé selon la revendication 8, comprenant une étape de pliage de la pièce de tissu (41, 42) selon un axe de pliage (AA', BB'), et des étapes de couture ou de soudure de bords (B1-B8, 51, 53, 55, 61, 63, 65) de la pièce de tissu (45, 46), pour former un dispositif de protection (45, 46) pouvant être enfilé sur une partie du corps humain.

10. Procédé selon l'une des revendications 8 et 9, pour la fabrication d'un dispositif de protection du pied, comprenant
- une étape de découpe dans la bande de tissu (23) d'une pièce de tissu (41) comprenant :
- quatre grandes branches (B1, B2, B3, B4), dont deux à gauche d'un axe de pliage (AA') et deux à droite de l'axe de pliage,
- entre deux grandes branches, deux petites branches tronquées (B5, B7) agencées à gauche et à droite de l'axe de pliage et orientées vers l'axe de pliage (AA'), comportant deux bords se rejoignant sur l'axe de pliage (AA'), et
- entre deux autres grandes branches, deux petites branches tronquées (B6, B8) agencées à gauche et à droite de l'axe de pliage et orientées vers l'axe de pliage, comportant deux bords se rejoignant sur l'axe de pliage,
- une étape de pliage de la pièce de tissu de manière que les petites branches se superposent, et
- une étape de couture ou de soudure des petites branches se superposant, pour former un module de protection du talon,
les grandes branches étant destinées à être reliées deux à deux par des moyens de liaison, pour former des bretelles de maintien du module de protection du talon.

11. Procédé selon l'une des revendications 8 et 9, pour la fabrication d'un dispositif de protection de la main, comprenant
- une étape de découpe dans la bande de tissu (23) d'une pièce de tissu (42) présentant deux parties (42A, 42B) symétriques relativement à un axe de pliage (BB'),
- une étape de pliage de la pièce de tissu (42) en sorte que les deux parties (42A, 42B) soient l'une au-dessus de l'autre, et
- une étape de couture ou de soudure pour relier deux à deux les bords (51, 61, 53, 63, 55, 65) se superposant des deux parties (42A, 42B), de sorte que le dispositif présente des bords non cousus ou non soudés formant des ouvertures permettant le passage du poignet et des doigts.

12. Procédé selon l'une des revendications 1 à 11, dans lequel la bande support (10) est en polychlorure de vinyle.

13. Procédé selon l'une des revendications 1 à 12, dans lequel les modules (21, 21') sont en gel silicone.

14. Procédé selon l'une des revendications 1 à 13, dans lequel le tissu (23) est du polyamide élasthanne.

## Patentansprüche

1. Verfahren zur kollektiven Herstellung einer Schutzvorrichtung (45, 46) umfassend ein Stück Stoff (41, 42) und ein Modul (21, 21') aus viskoelastischem Gel, das an dem Stoffstück angeklebt ist, **dadurch gekennzeichnet, dass** es die folgenden Schritte umfasst, die darin bestehen:
- eine Vielzahl von Alveolen (11) in einem Trägerstreifen (10) aus einem verformbaren Material zu bilden,
- die Alveolen mit einer unvernetzten Verbindung (20) zu füllen,
- die Verbindung (20) zu vernetzen, um in den Alveolen Module (21, 21') aus viskoelastischem Gel zu erhalten,
- auf jedes Modul (21, 21') eine klebende Substanz (22) aufzubringen und
- auf den Trägerstreifen (10) einen Stoffstreifen (23) aufzulegen, um einen fertig zusammengesetzten Streifen (1023) zu erhalten, in dem die in den Alveolen (11) vorhandenen Module (21, 21') aus viskoelastischem Gel durch die klebende Substanz (22) an dem Stoffstreifen (23) angeklebt sind.

2. Verfahren nach Anspruch 1, das zwischen den Schritten der Bildung der Module (21, 21') und des Aufbringens der klebenden Substanz (22) auf die Module (21) die folgenden Schritte umfasst, die darin bestehen:
- auf den Trägerstreifen (10) einen Schutzfilm (15) zu legen, um einen zusammengesetzten Zwischenstreifen (1015) zu erhalten, in dem die in den Alveolen (11) vorhandenen Module (21, 21') aus viskoelastischem Gel von dem Schutzfilm (15) bedeckt sind,
- den zusammengesetzten Zwischenstreifen (1015) aufzurollen, so dass eine Rolle (R2) aus zusammengesetztem Zwischenstreifen gebildet wird.

3. Verfahren nach Anspruch 2, in dem der Schutzfilm (15) auf den Trägerstreifen gelegt wird, bevor die Verbindung (20) in den Alveolen vollständig vernetzt ist und umfassend einen Schritt der Lagerung der Rolle (R2) aus zusammengesetztem Zwischenstreifen (1015), bei dem sich die Vernetzung der Verbindung (20) fortsetzen kann.

4. Verfahren nach einem der Ansprüche 2 und 3, das vor dem Schritt des Aufbringens der klebenden Substanz (22) auf die Module (21, 21') aus viskoelastischem Gel, einen Schritt umfasst, der darin besteht, die Rolle (R2) aus zusammengesetztem Zwischenstreifen (1015) zu entrollen und den Schutzfilm (15) abzuziehen.

5. Verfahren nach einem der Ansprüche 1 bis 4, umfassend einen Schritt, der darin besteht, eine Rolle (R3) aus dem fertig zusammengesetzten Streifen (1023) zu bilden.

6. Verfahren nach Anspruch 5, in dem die klebende Substanz (22) ein Polymerisationsklebstoff ist und umfassend einen Schritt der Lagerung der Rolle (R3) aus fertig zusammengesetztem Streifen, bei dem die klebende Substanz polymerisiert oder weiter polymerisiert.

7. Verfahren nach einem der Ansprüche 1 bis 6, umfassend einen Schritt, der darin besteht, den Trägerstreifen (10) vom Stoffstreifen (23) zu trennen, ohne dass die Module (21, 21') aus viskoelastischem Gel sich von dem Stoffstreifen lösen.

8. Verfahren nach Anspruch 7, umfassend einen Schritt des Zuschneidens des Stoffstreifens, um ein Stoffstück (41, 42) zu erhalten, das wenigstens ein Modul (21, 21') aus viskoelastischem Gel umfasst.

9. Verfahren nach Anspruch 8, umfassend einen Schritt, der darin besteht, das Stoffstück (41, 42) um eine Faltachse (AA', BB') zu falten, und Schritte, die darin bestehen, Ränder (B1-B8, 51, 53, 55, 61, 63, 65) des Stoffstücks (45, 46) zu vernähen oder zu verschweißen, um eine Schutzvorrichtung (45, 46) zu bilden, die auf einen menschlichen Körperteil gezogen werden kann.

10. Verfahren nach einem der Ansprüche 8 und 9, zur Fertigung einer Schutzvorrichtung für den Fuß, umfassend
- einen Schritt, der darin besteht, aus dem Stoffstreifen (23) ein Stoffstück (41) zuzuschneiden, das folgendes umfasst:
- vier große Arme (B1, B2, B3, B4), von denen sich zwei links der Faltachse (AA') und zwei rechts der Faltachse befinden,
- zwischen zwei großen Armen, zwei kleine stumpfe Arme (B5, B7), die links und rechts der Faltachse (AA') angeordnet und auf diese ausgerichtet sind und die zwei Ränder umfassen, die sich auf der Faltachse (AA') treffen,
- zwischen zwei weiteren großen Armen, zwei kleine stumpfe Arme (B6, B8), die links und rechts der Faltachse angeordnet und auf diese ausgerichtet sind und die zwei sich auf der Faltachse treffende Ränder umfassen.
- einen Schritt, der darin besteht, das Stoffstück so zu falten, dass sich die kleinen Arme überlagern, und
- einen Schritt, der darin besteht, die kleinen sich überlagernden Arme zu vernähen oder zu verschweißen, um ein Schutzmodul für die Ferse zu bilden,
wobei die großen Arme dazu bestimmt sind, jeweils paarweise durch Verbindungsmittel miteinander verbunden zu werden, um Halteträger für das Schutzmodul der Ferse zu bilden.

11. Verfahren nach einem der Ansprüche 8 und 9, zur Fertigung einer Schutzvorrichtung für die Hand, umfassend:
- einen Schritt, der darin besteht, aus dem Stoffstreifen (23) ein Stoffstück (42) zuzuschneiden, das zwei in Bezug auf eine Faltachse (BB') symmetrische Bereiche (42A, 42B) aufweist,
- einen Schritt, der darin besteht, das Stoffstück (42) so zu falten, dass die beiden Bereiche (42A, 42B) übereinanderliegen, und
- einen Schritt, der darin besteht, die jeweils paarweise sich überlagernden Ränder (51, 61, 53, 63, 55, 65) der beiden Bereiche (42A, 42B) zusammenzunähen oder
- zuschweißen, so dass die Vorrichtung nicht zusammengenähte oder nicht zusammengeschweißte Ränder aufweist, die Öffnungen bilden, durch die das Handgelenk und die Finger durchpassen.

12. Verfahren nach einem der Ansprüche 1 bis 11, in dem der Trägerstreifen (10) aus Vinylpolychlorid besteht.

13. Verfahren nach einem der Ansprüche 1 bis 12, in dem die Module (21, 21') aus Silicongel bestehen.

14. Verfahren nach einem der Ansprüche 1 bis 13, in dem der Stoff (23) aus Polyamid Elasthan besteht.

## Claims

1. A method for collectively manufacturing a protection device (45, 46) comprising a piece of fabric (41, 42) and a module (21, 21') made of viscoelastic gel glued onto the piece of fabric, **characterized in that** it comprises the steps of:
- forming a plurality of cells (11) in a support strip (10) made of a deformable material,
- filling the cells with a non-cross-linked compound (20),
- cross-linking the compound (20) to obtain modules (21, 21') made of viscoelastic gel in the cells,
- depositing on each module (21, 21') an adhesive substance (22), and
- depositing a strip of fabric (23) on the support strip (10) to obtain a final compound strip (1023) in which the modules (21, 21') made of viscoelastic gel present in the cells (11) are glued to the strip of fabric (23) by the adhesive substance (22).

2. Method according to claim 1 comprising, between the steps of forming the modules (21, 21') and of depositing an adhesive substance (22) on the modules (21), the steps of:
- depositing a protective film (15) on the support strip (10) to obtain an intermediate compound strip (1015) in which the modules (21, 21') made of viscoelastic gel present in the cells (11) are covered by the protective film (15),
- winding the intermediate compound strip (1015) so as to form a roll (R2) of intermediate compound strip.

3. Method according to claim 2, wherein the protective film (15) is deposited on the support strip before the compound (20) in the cells is entirely cross-linked, and comprising a step of storing the roll (R2) of intermediate compound strip (1015) during which the cross-linking of the compound (20) can continue.

4. Method according to one of claims 2 and 3 comprising, before the step of depositing the adhesive substance (22) on the modules (21, 21') made of viscoelastic gel, a step of unwinding the roll (R2) of intermediate compound strip (1015) and of removing the protective film (15).

5. Method according to one of claims 1 to 4, comprising a step of forming a roll (R3) with the final compound strip (1023).

6. Method according to claim 5, wherein the adhesive substance (22) is a polymerizable glue, and comprising a step of storing the roll (R3) of final compound strip during which the adhesive substance polymerizes or continues to polymerize.

7. Method according to one of claims 1 to 6, comprising a step of separating the support strip (10) from the strip of fabric (23) without unsticking the modules (21, 21') made of viscoelastic gel from the strip of fabric.

8. Method according to claim 7, comprising a step of cutting the strip of fabric to obtain a piece of fabric (41, 42) comprising at least one module (21, 21') made of viscoelastic gel.

9. Method according to claim 8, comprising a step of folding the piece of fabric (41, 42) according to a folding axis (AA', BB'), and steps of sewing or binding edges (B1-B8, 51, 53, 55, 61, 63, 65) of the piece of fabric (45, 46) to form a protection device (45, 46) which can be slipped onto a part of the human body.

10. Method according to one of claims 8 and 9 for manufacturing a protection device for protecting the foot, comprising
- a step of cutting out of the strip of fabric (23) a piece of fabric (41) comprising:
- four large branches (B1, B2, B3, B4), including two on the left of a folding axis (AA') and two on the right of the folding axis,
- between two large branches, two small truncated branches (B5, B7) arranged on the left and right of the folding axis and towards the folding axis (AA'), comprising two edges joining on the folding axis (AA'), and
- between two other large branches, two small truncated branches (B6, B8) arranged on the left and right of the folding axis and towards the folding axis, comprising two edges joining on the folding axis,
- a step of folding the piece of fabric so that the small branches are superimposed, and
- a step of sewing or binding the small superimposed branches, to form a module for protecting the heel,
the large branches being intended to be linked in pairs by connection means, to form straps for holding the heel protection module.

11. Method according to one of claims 8 and 9 for manufacturing a protection device for protecting the hand, comprising
- a step of cutting out of the strip of fabric (23) a piece of fabric (42) having two symmetrical parts (42A, 42B) in relation to a folding axis (BB'),
- a step of folding the piece of fabric (42) so that the two parts (42A, 42B) are one above the other, and
- a step of sewing or binding to link in pairs the superimposed edges (51, 61, 53, 63, 55, 65) of the two parts (42A, 42B), such that the device has edges not sewn or bound forming openings enabling the wrist and fingers to be passed through it.

12. Method according to one of claims 1 to 11, wherein the support strip (10) is made of polyvinyl chloride.

13. Method according to one of claims 1 to 12, wherein the modules (21, 21') are made of silicone gel.

14. Method according to one of claims 1 to 13, wherein the fabric (23) is elastane polyamide.
